# EUROPÄISCHE PATENTANMELDUNG

(11) **EP 0 770 379 A2**
(43) Veröffentlichungstag der Anmeldung: **02.05.1997**
(21) Anmeldenummer: 96202853.6
(22) Anmeldetag: 02.10.1996
(51) Int. Cl.: A61K 7/48

(54) **Emulgatorfreie, feindisperse kosmetische oder dermatologische Zubereitungen vom Typ Wasser-in-Öl**

(30) Priorität: 24.10.1995 DE 19539428
(71) Anmelder: Beiersdorf Aktiengesellschaft, D-20245 Hamburg (DE)
(72) Erfinder: Schönrock, Uwe, Dr., 22851 Norderstedt (DE); Christiansen, Michael, 25436 Tornesch (DE); Steinke, Sigrid, 21075 Hamburg (DE)

(57) **Zusammenfassung**

Feindisperse, emulgatorfreie kosmetische oder dermatologische Zubereitungen vom Typ Wasser-in-Öl, enthaltend
- eine Ölphase, enthaltend als Hauptbestandteil ein oder mehrere unpolare Öle, Fette und/oder Wachse
- eine Wasserphase
- ein oder mehrere Salze aus zwei- und/oder dreiwertigen Metallkationen und einer oder mehreren Alkylcarbonsäuren mit 10 - 24 C-Atomen,
- sowie gewünschtenfalls enthaltend übliche kosmetische oder dermatologische Hilfs-, Zusatz- und/oder Wirkstoffe.

## Beschreibung

Die vorliegende Erfindung betrifft kosmetische und dermatologische Zubereitungen vom Typ Wasser-in-öl, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, emulgatorfreie Sonnenschutzprodukte zu entwickeln.

Emulgatorfreie Lichtschutzpräparate auf Basis sogenannter Hydrodispersionen sind seit einiger Zeit für den Verbraucher zugängig.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

W/O-Lipodispersionen, welche den Gegenstand der vorliegenden Erfindung darstellen, sind in umgekehrter Analogie emulgatorfreie feindisperse Zubereitungen vom Typ Wasser-in-Öl.

Zubereitungen des Standes der Technik haben im allgemeinen den Nachteil, daß sie entweder instabil sind, auf einen engen Anwendungsbereich oder eine begrenzte Einsatzstoffauswahl begrenzt sind. Meist verhält es sich darüberhinaus so, daß Zusammensetzungen des Standes der Technik wenig oder gar keine hautpflegende Fette oder Öle enthalten.

Eine Aufgabe der vorliegenden Erfindung war es, emulgatorfreie feindisperse Zubereitungen vom Typ Wasser-in-Öl herzustellen, welche nicht die Nachteile des Standes der Technik aufweisen. Eine weitere Aufgabe der Erfindung war, das begrenzte Angebot an emulgatorfreien feindispersen Zubereitungen vom Typ Wasser-in-Öl des Standes der Technik zu bereichern. Ferner war es Aufgabe der vorliegenden Erfindung, stabile, emulgatorfreie Zubereitungen mit hohem Fett- und/oder Ölanteil zur Verfügung zu stellen.

Erstaunlicherweise werden diese Aufgaben gelöst durch feindisperse, emulgatorfreie kosmetische oder dermatologische Zubereitungen vom Typ Wasser-in-Öl, enthaltend
- eine Ölphase, enthaltend als Hauptbestandteil ein oder mehrere unpolare Öle, Fette und/oder Wachse
- eine Wasserphase
- ein oder mehrere Salze aus zwei- und/oder dreiwertigen Metallkationen und einer oder mehreren Alkylcarbonsäuren mit 10 - 24 C-Atomen,
- sowie gewünschtenfalls enthaltend übliche kosmetische oder dermatologische Hilfs-, Zusatz- und/oder Wirkstoffe.

Erfindungsgemäß vorteilhaftes Salz aus zwei- und/oder dreiwertigen Metallkationen und einer oder mehreren Alkylcarbonsäuren mit 10 - 24 C-Atomen sind die Aluminiumstearate.

Vorteilhaft werden die erfindungsgemäßen unpolaren Fette, Wachse bzw. Öle gewählt aus der Gruppe Vaseline (Petrolatum), Paraffinöl, Polyolefine. Unter den Polyolefinen sind Polydecene die bevorzugten Substanzen.

Ferner ist es möglich und vorteilhaft, einen zusätzlichen Gehalt an Silikonölen in die erfindungsgemäßen Zubereitungen einzuarbeiten..

Vorteilhaft enthalten erfindungsgemäße Zubereitungen 0,5 - 50 Gew.-% eines oder mehrerer unpolarer Öle, Fette und/oder Wachse, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Besonders vorteilhaft enthalten erfindungsgemäße Zubereitungen 0,5 - 50 Gew.-% Petrolatum, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Auch besonders vorteilhaft enthalten erfindungsgemäße Zubereitungen 0,5 - 50 Gew.-% Paraffinöl, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Ganz besonders vorteilhaft enthalten erfindungsgemäße Zubereitungen 0,5 - 30 Gew.-% Petrolatum, 0,5 - 30 Gew.-% Paraffinöl, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Bevorzugt enthalten erfindungsgemäße Zubereitungen 1,5 - 20 Gew.-% Petrolatum, 3 - 18 Gew.-% Paraffinöl, 0,1 - 2 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Besonders bevorzugt enthalten erfindungsgemäße Zubereitungen 5 - 10 Gew.-% Petrolatum, 8 - 18 Gew.-% Paraffinöl, 0,1 - 2 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.Kombination.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Die erfindungsgemäßen kosmetischen und dermatologischen Zubereitungen können ferner kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, Verdikkungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Die nachfolgenden Beispiele sollen die vorliegende Erfindung verdeutlichen.

| **Beispiel 1** | |
|---|---|
| | Gew.-% |
| Dimethicon | 2,00 |
| Phenyltrimethicon | 4,00 |
| Petrolatum DAB 10 | 10,00 |
| Polydecen | 30,00 |
| C₁₂₋₁₅-Alkylbenzoat | 3,00 |
| Octoxyglycerin | 1,00 |
| Butylenglycol | 5,00 |
| Aluminiumstearat | 0,80 |
| Behenylalkohol | 4,00 |
| Hydriertes Rizinusöl | 4,00 |
| Bisabolol | 0,10 |
| Wasser, demin. | ad 100,00 |

## Patentansprüche

1. Feindisperse, emulgatorfreie kosmetische oder dermatologische Zubereitungen vom Typ Wasser-in-Öl, enthaltend
- eine Ölphase, enthaltend als Hauptbestandteil ein oder mehrere unpolare Öle, Fette und/oder Wachse
- eine Wasserphase
- ein oder mehrere Salze aus zwei- und/oder dreiwertigen Metallkationen und einer oder mehreren Alkylcarbonsäuren mit 10 - 24 C-Atomen,
- sowie gewünschtenfalls enthaltend übliche kosmetische oder dermatologische Hilfs-, Zusatz- und/oder Wirkstoffe.

2. Zubereitungen nach Anspruch 1, dadurch gekennzeichnet, daß als Salz oder Salze aus zwei- und/oder dreiwertigen Metallkationen und einer oder mehreren Alkylcarbonsäuren mit 10 - 24 C-Atomen ein oder mehrere Aluminiumstearate gewählt werden.

3. Zubereitungen nach Anspruch 1, dadurch gekennzeichnet, daß die Öle, Fette bzw. Wachse gewählt werden aus der Gruppe Vaseline (Petrolatum), Paraffinöl, Polyolefine.

4. Zubereitungen nach Anspruch 1, enthaltend 0,5 - 50 Gew.-% eines oder mehrerer unpolarer Öle, Fette und/oder Wachse, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

5. Zubereitungen nach Anspruch 1, enthaltend 0,5 - 50 Gew.-% Petrolatum, 0,005 - 10 Gew.-% eines oder mehrerer Salze aus zwei- und/oder dreiwertigen Metallkationen und einer oder mehreren Alkylcarbonsäuren mit 10 - 24 C-Atomen, gegebenenfalls Hilfs-, Zusatz- oder Wirkstoffe sowie Wasser.

6. Verwendung von Zubereitungen nach Anspruch 1 als kosmetisches oder dermatologisches Vehikel zum Transport von Wirkstoffen, insbesondere von Antioxidantien, in die Haut.
